# NEUE EUROPÄISCHE PATENTSCHRIFT

(11) **EP 1 066 050 B2**
(45) Veröffentlichungstag und Bekanntmachung des Hinweises auf die Entscheidung über den Einspruch: **02.06.2010**
(45) Hinweis auf die Patenterteilung: 15.06.2005
(21) Anmeldenummer: 99913314.3
(22) Anmeldetag: 29.03.1999
(51) Int. Cl.: A61K 38/17, A61K 35/26, A61P 35/00, A61P 37/04

(54) **VERWENDUNG VON HSP70 PROTEIN**
APPLICATION OF HSP70 PROTEINS
UTILISATION DE PROTEINES HSP70

(30) Priorität: 27.03.1998 DE 19813760; 26.03.1999 WO PCT/EP99/02056
(43) Veröffentlichungstag der Anmeldung: 10.01.2001
(73) Patentinhaber: Multhoff, Gabriele, Prof. Dr., 81675 München (DE)
(72) Erfinder: Multhoff, Gabriele, Prof. Dr., 81675 München (DE)
(74) Vertreter: Steinecke, Peter
(86) Internationale Anmeldenummer: PCT/EP1999/002165
(87) Internationale Veröffentlichungsnummer: WO 1999/049881

(56) Entgegenhaltungen:
- EP-A- 0 843 005
- EP-B1- 0 584 715
- WO-A1-91//15219
- WO-A1-93//31529
- WO-A2-95//03418
- US- - 5 348 945
- S. SUZUKI ET AL.: "IL-12 induced enhancement of MHC class I antigen expression on cancer cells." PROCEEDINGS OF THE AMERICAN ASSOCIATION FOR CANCER RESEARCH, Bd. 37, März 1996 (1996-03), Seite 445 XP002118456 USA
- Y. TAMURA ET AL.: "Immunotherapy of tumors with autologous tumor-derived heat shock protein preparations." SCIENCE, Bd. 278, Nr. 5335, 3. Oktober 1997 (1997-10-03), Seiten 117-120, XP002118457 Washington, DC, USA in der Anmeldung erwähnt
- G. MULTHOFF ET AL.: "CD3- large granular lymphocytes recognize a heat-inducible immunogenic determinant associated with the 72-kD heat shock protein on human sarcoma cells." BLOOD, Bd. 86, Nr. 4, 15. August 1995 (1995-08-15), Seiten 1374-1382, XP002090860 New York, NY, USA
- C. SAVARY ET AL.: "Role of heat shock proteins (Hsp) in immunity to breast cancer." BREAST CANCER RESEARCH AND TREATMENT, Bd. 46, Nr. 1, Oktober 1997 (1997-10), Seite 69 XP002118458 Den Haag, Niederlande
- G. MULTHOFF ET AL.: "Heat shock proteins and the immune response." ANNALS OF THE NEW YORK ACADEMY OF SCIENCES, Bd. 851, 1998, Seiten 86-93, XP002118459 New York, NY, USA
- H. UDONO ET AL.: "Comparison of tumor-specific immunogenicities of stress-induced proteins gp96, hsp90, and hsp70." THE JOURNAL OF IMMUNOLOGY, Bd. 152, Nr. 11, 1. Juni 1994 (1994-06-01), Seiten 5398-5403, XP002118460 Baltimore, MD, USA
- C. BOTZLER ET AL.: "Definition of extracellular localized epitopes of Hsp70 involved in an NK immune response." CELL STRESS AND CHAPERONES, Bd. 3, Nr. 1, März 1998 (1998-03), Seiten 6-11, XP002118461 in der Anmeldung erwähnt
- G. MULTHOFF ET AL.: "The role of heat shock proteins in the stimulation of an immune response." BIOLOGICAL CHEMISTRY, Bd. 379, Nr. 3, März 1998 (1998-03), Seiten 295-300, XP002118462 in der Anmeldung erwähnt
- IMMUNOLOGICAL INVESTIGATIONS Bd. 29, Nr. 2, 2000, Seiten 131 - 137
- Internet homepage www.multiimmune.de

## Beschreibung

Die Erfindung betrifft die Verwendung von Hsp70-Protein, das nicht mit Peptiden aus Tumoren komplexiert ist, oder Fragmenten davon zur Aktivierung von NK-Zellen, Arzneimittel, Medizinprodukte oder medizinische Hilfsstoffe, die Fragmente oder Derivate eines solchen Hsp70-Proteins oder aktivierte NK-Zellen enthalten, zur Behandlung von Tumoren, Krebserkrankungen oder Infektionskrankheiten, Verfahren zur Aktivierung von NK-Zellen, sowie medizinische Verwendungen der durch das erfindungsgemäße Verfahren erhaltenen Produkte.

Chaperone sind für eine Anzahl fundamentaler Prozesse in der Zelle notwendig. Insbesondere ist bekannt, daß sie dem Zellstreß entgegenwirken. Die am besten untersuchte Klasse von Chaperonen ist die Gruppe der Hitzeschock-Proteine (HSP) mit einem Molekulargewicht von 70 kDa (Multhoff et al., Cell Stress & Chaperones 1 (3) (1996), 167). Diese Proteine sind evolutionär hochkonserviert. Sie binden intrazellulär an nicht gefaltete oder nicht korrekt gefaltete Polypeptide, stabilisieren sie und inhibieren auf diese Weise deren Aggregation oder ermöglichen eine Transmembran-Translokation. Hsp70 ist sowohl im Zellkern, im Zytosol und an der Zelloberfläche bestimmter Tumorzellen lokalisiert. Neben ihrer intrazellulären Aufgabe als Chaperone scheinen die Mitglieder der HSP70-Familie an der Stimulierung des Immunsystems beteiligt zu sein, z. B. entzündlicher Prozesse unter Beteiligung von Pathogenen und an der zellulären anti-Tumorimmunantwort in vivo und in vitro. Entsprechend sind im Stand der Technik therapeutische Verwendungsmöglichkeiten von Hitzeschockproteinen dargestellt worden. So wird in der WO 97/10000 der Einsatz von Komplexen, die aus einem Hitzeschockprotein und einem an dieses Protein nicht-kovalent gebundenem, exogenem Antigenmolekül (Peptid) bestehen, zur Prävention und Behandlung von Tumor- und Infektionserkrankungen beschrieben. Die mit Hitzeschockproteinen im Komplex vorliegenden Antigene stammen aus Tumorzellen. Sie weisen die gemeinsame Eigenschaft auf, eine Immunantwort zu induzieren. Multhoff et al., Biol. Chem. 379 (1998), 295-300 ordnen HSPs, darunter Hsp70, eine Rolle in der Erkennung durch nicht-MHC-restringierte Effektorzellen, darunter NK-Zellen, zu. Insbesondere wird herausgestellt, daß NK-Zellen auf der Oberfläche von Tumorzellen präsentierte Hsp70-Moleküle erkennen und die Tumorzellen sodann lysieren. Ein weiterer Ansatz zur Therapie von Krebserkrankungen wurde von Tamura und Kollegen (Tamura et al., Science 278 (1997), 117-123) vorgestellt. Sie konnten belegen, daß Tumor-tragende Mäuse dann erfolgreich mit Hitzeschockproteinpräparationen behandelt werden können, wenn diese aus autologen Tumoren stammen. Präparationen aus nicht-autologen Tumoren oder aus Normalgewebe hingegen führen nicht zur Regression der Tumore (Blachere et al., J. Exp. Med. 186 (1997), 1315-1322). Die HSPs sind in der Studie von Tamura et al. mit einer Vielzahl nicht näher identifizierter Peptide komplexiert. Zusammenfassend kann festgehalten werden, daß der Stand der Technik eine immunologische Aktivität von HSP-Molekülen belegt, wenn diese entweder mit Peptiden komplexiert sind und/oder auf der Oberfläche von Zellen wie Tumorzellen präsentiert werden. Obwohl somit das Potential von Hitzeschockproteinen bei der Bekämpfung unterschiedlicher Erkrankungen in erster Näherung erkannt ist, hängt der erfolgreiche therapeutische Einsatz in der Regel jedoch von der Präparation bestimmter Komplexe oder Zellaufbereitungen sowie von der Menge des Ausgangsmaterials (Tumormaterials) ab. Ein universeller, patientenunabhängiger Einsatz dieser Komplexe oder Zellaufbereitungen ist nur schwer vorstellbar.

Aufgabe der vorliegenden Erfindung war demzufolge, neue Mittel und Wege für die Nutzung des immunologischen Potentials von Hitzeschockproteinen bereitzustellen, die unbelastet von den vorstehend genannten aus dem Stand der Technik bekannten Nachteilen sind.

Diese Aufgabe wird durch die in den Ansprüchen dargestellten Ausführungsformen gelöst.

Somit betrifft die Erfindung die Verwendung eines Hsp70-Proteins, das nicht mit Peptiden aus Tumorzellen komplexiert ist, eines carboxy-terminalen (C-terminalen) Fragmentes davon oder eines Derivats davon oder eines Proteins mit einer Aminosäuresequenzhomologie zum C-terminalen Bereich des Hsp70-Proteins von ≥ 70% zur Herstellung eines Arzneimittels, Medizinproduktes oder medizinischen Hilfsstoffes zur Behandlung von Tumoren, Krebserkrankungen oder Infektionskrankheiten durch Aktivierung von NK-Zellen.

Arzneimittel sind erfindungsgemäß als Stoffe und Zubereitungen aus Stoffen definiert, die dazu bestimmt sind, durch Anwendung am oder im menschlichen Körper Krankheiten, Leiden, Körperschäden oder krankhafte Beschwerden zu heilen, zu lindern, zu verhüten oder zu erkennen.

Medizinprodukte sind erfindungsgemäß alle einzeln oder miteinander verbunden verwendeten Stoffe und Zubereitungen aus Stoffen oder andere Gegenstände, die vom Hersteller zur Anwendung für Menschen mittels ihrer Funktionen zum Zwecke der Erkennung, Verhütung, Überwachung, Behandlung oder Linderung von Krankheiten zu dienen bestimmt sind und deren bestimmungsgemäße Hauptwirkung im oder am menschlichen Körper weder durch pharmakologisch oder immunologisch wirkende Mittel noch durch Metabolismus erreicht wird, deren Wirkungsweise aber durch solche Mittel unterstützt werden kann.

Medizinische Hilfsstoffe sind erfindungsgemäß solche Stoffe, die zur Produktion (als aktive Ingredienzien) von Arzneimitteln eingesetzt werden.

Ferner betrifft die Erfindung die Verwendung eines Hsp70-Proteins, eines C-terminalen Fragmentes davon oder eines Derivats davon oder eines Proteins mit einer Aminosäuresequenzhomologie zum C-terminalen Bereich (Aminosäuren 384-641) des Hsp70-Proteins von ≥ 70% zur ex vivo oder in vitro Aktivierung von NK-Zellen.

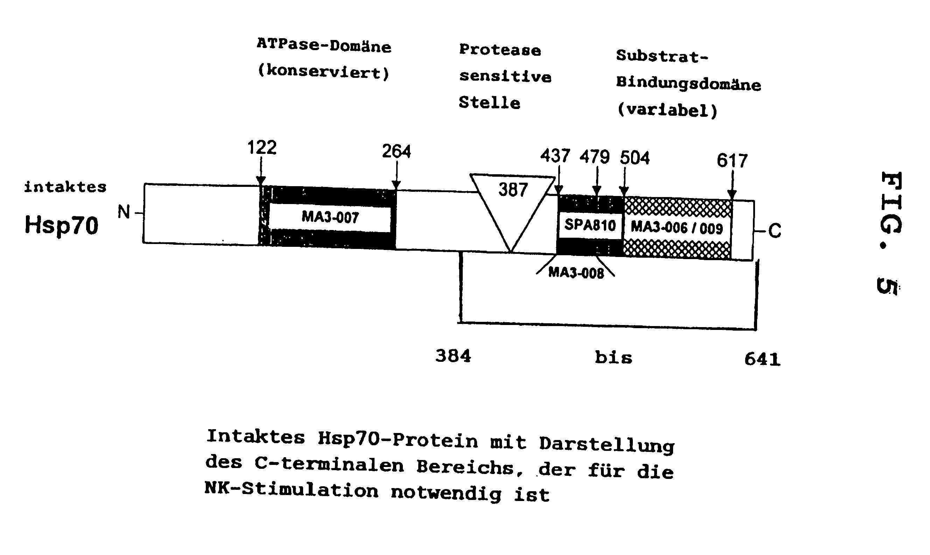

Der Begriff "Hsp70-Protein" erfaßt erfindungsgemäß eukaryontische Hitzeschockproteine, deren Expression durch Hitze, aber auch durch eine Vielzahl anderer Reagenzien wie z.B. Aminosäure-Analoga, Schwermetalle, Ionophore oder Zellgifte induzierbar ist, wobei der Faktor der Steigerung der Expression durch die Induktion im Verhältnis zur konstitutiven Expression mindestens 5 beträgt. Die beiliegende Figur 5 zeigt den Aufbau eines Hsp70-Proteins bestehend aus einer N-terminalen ATPase Domäne und einer C-terminalen Substrat-Bindungsdomäne des Proteins. Die vollständige Aminosäuresequenz ist veröffentlicht in Milner, et al., Immunogenetics 32 (4) (1990), 242-251.

Der Begriff "carboxy-terminale[s] (C-terminale[s]) Fragment" des Hsp70-Proteins umfaßt erfindungsgemäß (Poly)peptide, die eine Aminosäuresequenz aus dem Bereich der Aminosäuren 384-641 des menschlichen Hsp70 aufweisen. Umfaßt von der vorliegenden Erfindung sind auch Fragmente des C-terminalen Fragmentes 384-641. In anderen Ausführungsformen sind mit diesem Begriff (Poly)peptide erfaßt, die aus dem Bereich eines anderen, von dem erfindungsgemäß verwendeten Begriff "Hsp70-Protein" erfaßten Proteins stammen, der zu dem genannten C-terminalen Bereich des menschlichem Hsp70-Proteins homolog ist. Die erfindungsgemäß verwendeten Fragmente des Hsp70-Proteins weisen ebenfalls die Fähigkeit auf, NK-Zellen zu aktivieren. Die Aktivierung kann vom Fachmann ohne weiteres anhand der Lehre der Erfindung überprüft werden. Insofern ist der Fachmann auch ohne weiteres in der Lage, Fragmente aus dem vorstehend genannten Fragment 384-641 gentechnologisch herzustellen (allgemeine Verfahren hierzu sind beschrieben in Sambrook et al., "Molecular Cloning, A Laboratory Manual", 2. Auflage 1989, CSH Press, Cold Spring Harbor, N. Y.) und auf die gewünschten Aktivierungseigenschaften hin zu testen.

Der Begriff "Derivat" umfaßt sowohl Derivate des Hsp70-Proteins als auch Derivate des C-terminalen Fragmentes, sofern diese Derivate die erfindungsgemäßen Funktionen aufweisen. Derartige Derivate weisen vorzugsweise dieselbe dreidimensionale Struktur wie Hsp-70 bzw. dessen C-terminale Fragmente auf und können beispielsweise durch Peptidomimetics hergestellt werden (al-Obeidi et al., Mol. Biotechnol. 9 (1998), 205-223; Wiley et al., Med. Res. Rev. 13 (1993), 327-384; Bohm, J. Comput. Aided Mol. Des. 10 (1996), 265-272; Hruby et al., Biopolymers 43 (1997) , 219-266).

Der Begriff "NK-Zellen" ("Natürliche Killerzellen", engl.: "natural killer cells") umfaßt große, granuläre Lymphozyten, die CD45 auf der Oberfläche exprimieren und ohne vorherige Stimulation Killeraktivität aufweisen. Sie sind insbesondere dadurch gekennzeichnet, daß sie CD16 exprimieren und/oder durch Interleukin-2 stimulierbar sind und/oder kein CD3 exprimieren und/oder keine α/β- oder γ/δ- T-Zell-Rezeptoren besitzen.

Die NK-Zellen, die im erfindungsgemäßen Verfahren ihre Wirksamkeit entfalten, sind weiterhin bevorzugt durch die nachfolgenden Eigenschaften gekennzeichnet:
- sie sind transient plastik-adhärent nach Zugabe von IL-2 in Mengen von 10 bis 10.000 Einheiten, z.B. von 100 1 E, wobei IL-2 von der Firma Chiron bezogen werden kann;
- die Adhärenz erfolgt 3-18 Stunden nach Zugabe des IL-2 auf frisch isolierte PBL (monozytendepletierte, periphere Blutlymphozyten);
- die NK-Zellen weisen eine CD16dim Expression auf (Mittelwert der Fluorszenz schwach);
- die NK-Zellen exprimieren CD56 und CD57 als typische NK-Marker;
- die NK-Zellen exprimieren CD94 (C-Typ Lectin Killer-Zell-Rezeptor);
- die NK-Zellen sezernieren nach Aktivierung mit Hsp70 und Zytokinen IFNgamma;
- die NK-Zellen sind durch Zugabe von Hsp70 (gereinigtes Protein) stimulierbar (Wachstum und zytotoxische Aktivität);
- sie sind nicht vom MHC-Typ des Patienten abhängig.

Erfindungsgemäß können auch andere NK-Zellpopulationen eingesetzt werden. Voraussetzung ist jedoch, daß sie durch das erfindungsgemäß eingesetzte Hsp70 oder durch die genannten Fragmente oder Derivate aktivierbar sind. Erfindungsgemäß können isolierte NK-Zellen eingesetzt werden. Es ist aber auch möglich, Zellgemische wie periphere mononukleäre Blutzellen (PBMC) einzusetzen, in denen NK-Zellen enthalten sind.

Der Begriff "Aminosäuresequenzhomologie zum C-terminalen Bereich des Hsp70-Proteins von ≥ 70%" bedeutet im Sinne dieser Erfindung, daß mindestens 70% der Aminosäuren bei einer Gegenüberstellung ("alignment") von zwei Aminosäuresequenzen identisch sind, wobei die eine der gegenübergestellten Aminosäuresequenzen die des C-terminalen Bereichs von Hsp70 ist. Umfaßt sind auch solche Sequenzen, bei denen 70% der Aminosäuren identisch sind, die sich aber zusätzlich von der C-terminalen Hsp70-Referenzsequenz bei der Gegenüberstellung durch Lücken ("gaps") unterscheiden. Diese Lücken können entweder im erfindungsgemäß eingesetzten homologen Molekül oder im Referenzmolekül auftreten. Alignments, üblicherweise durch Computervergleich, sind im Stand der Technik bekannt, desgleichen die Programme, mit denen derartige Alignments durchgeführt werden können. Es ist außerdem bevorzugt, daß die Proteine bzw. Fragmente eine Aminosäuresequenzhomologie zum carboxy-terminalen Bereich, also im Bereich der Aminosäuren 384 - 641, des Hsp70-Proteins von ≥ 80% und bevorzugt ≥ 90%, aufweisen.

Der Befund, daß Hitzeschockproteine, C-terminale Fragmente davon oder davon abgeleitete Derivate über die Aktivierung von NK-Zellen immunologische Aktivitäten induzieren, selbst wenn sie nicht mit Peptiden komplexiert sind oder auf der Oberfläche von Zellen wie Tumorzellen präsentiert werden, muß als höchst überraschend angesehen werden. Beispielsweise ging man noch im Juni 1998 (vgl. Srivastava et al., Immunity 8 (1998), 657-665, hier gutachtlich zitiert) davon aus, daß isolierte Hitzeschockproteine keine immunogenen Wirkungen wie CTL-Induktion aufweisen (Blachere et al., J. Exp. Med. 186 (1997), 1315-1322) und keine protektive Immunität gegenüber irgendeiner Krebsart induzieren können (Udono und Srivstava, J. Immunol. 152 (1994), 5398-5403). Mit der vorliegenden Erfindung wird hingegen möglich, patientenunabhängig eine in vitro oder in vivo Aktivierung von NK-Zellen herbeizuführen, die nach der Aktivierung erfolgreich gegen verschiedene Krankheiten, beispielsweise Tumorerkrankungen eingesetzt werden können. Die Verwendung von isoliertem Hitzeschockprotein erlaubt darüber hinaus eine bessere Standardisierung von Aktivierungsvorgänaen. Mit der vorliegenden Erfindung ist es möglich, HSP in unbegrenzter Menge herzustellen, wohingegen bei der patientenspezifischen Aufbereitung der HSP-Peptid Komplexe die Menge an HSP über die Menge des Tumors limitiert ist.

Überraschend ist über die vorgenannten Befunde hinaus, daß auch der Einsatz von C-terminalen Fragmenten des Hsp70-Proteins zum erfindungsgemäßen Ergebnis führt. Unerwartet ist vor allem, daß der C-Terminus offensichtlich dieselbe dreidimensionale Struktur aufweist, die von NK-Zellen erkannt wird und zu deren Aktivierung führt. Die Möglichkeit des Einsatzes von C-terminalen Hsp70-Fragmenten in der Aktivierung von NK-Zellen birgt unter anderem den Vorteil, daß die rekombinante Herstellung zu besseren Ausbeuten im Vergleich zur rekombinanten Darstellung des gesamten Proteins führen sollte.

Die Herstellung von Derivaten von Hsp70 oder dessen C-terminalen Fragmenten, beispielsweise durch Peptidomimetics, ist beispielsweise dann sinnvoll, wenn der rasche Abbau dieser (Poly)peptide im Körper vermieden werden soll. Dies kann z. B. bei der oralen Gabe von Arzneimitteln eine Rolle spielen.

Vorzugsweise umfaßt die Aktivierung die Induktion einer durch NK-Zellen vermittelten Immunantwort. Dabei ist besonders bevorzugt, daß die durch NK-Zellen vermittelte Immunantwort eine Stimulation der Proliferation der NK-Zellen und/oder die Steigerung der zytolytischen Aktivität der NK-Zellen umfaßt. Der Einsatz von Hsp70 bzw. von Fragmenten oder Derivaten davon erfolgt in allen vorgenannten Ausführungsformen in einer (pharmazeutisch) wirksamen Menge, so daß die gewünschte Aktivierung, vorzugsweise die Immunantwort, induziert wird. Die Immunantwort richtet sich vornehmlich, aber nicht ausschließlich gegen solche Zellen, die Hsp70 oder Fragmente davon auf der Zelloberfläche exprimieren. Hierzu gehören sowohl menschliche als auch tierische Zellen. Zu diesen Hsp70 auf der Zelloberfläche exprimierenden menschlichen oder tierischen Zellen gehören beispielsweise Tumorzellen und Zellen von Patienten mit Infektionskrankheiten. Die zytolytische Aktivität der durch Hsp70 erfindungsgemäß stimulierten NK-Zellen ist signifikant erhöht, so daß eine immunologische Elimination dieser Hsp70 auf der Zelloberfläche exprimierenden Zellen ermöglicht wird.

In einer besonders bevorzugten Ausführungsform der erfindungsgemäßen Verwendung ist die zytolytische Aktivität gegenüber Tumorzellen und/oder Zellen von Patienten mit Infektionserkrankungen gesteigert.

In einer weiteren besonders bevorzugten Ausführungsform der erfindungsgemäßen Verwendung ist die zytolytische Aktivität gegenüber Leukämiezellen, Lymphomzellen, Tumorzellen und metastasierenden Zellen solider Tumore und Zellen von Patienten mit viralen, mykotischen oder bakteriellen Infektionserkrankungen gesteigert. Ein Beispiel für die Behandlung viraler Erkrankungen ist die Behandlung von HIV-Infektionen, ein Beispiel für eine bakterielle Infektion ist die Behandlung von durch Mykobakterien hervorgerufenen Erkrankungen. Zu den soliden Tumoren, deren Metastasenzellen durch das erfindungsgemäße immunologische Verfahren behandelbar sind, gehören beispielsweise Karzinome, Sarkome, Melanome oder Leukämien und Lymphome. Beispiele für Karzinome sind Kolonkarzinome und Lungenkarzinome.

Durch die erfindungsgemäße Verwendung eines Hsp70-Proteins, Teilen dieses Proteins oder ≥ 70% sequenzhomologer Proteine können Zellen lysiert werden, die durch Viren, Bakterien und/oder Pilze infiziert sind oder Zellen, die tumorigen verändert sind. Auch Zellen, die antigene Teile dieser Fremdorganismen oder Teile von Tumorzellen enthalten, können durch die erfindungsgemäße Anwendung des Hsp70-Proteins mit Hilfe der aktivierten NK-Zellen lysiert werden.

Die Erfindung betrifft ferner ein Verfahren zur ex vivo oder in vitro Aktivierung von NK-Zellen, wobei man eine NK-Zellen enthaltende physiologische Zellsuspension mit einem Hsp70-Protein, einem C-terminalen Fragment davon oder einem Derivat davon oder einem Protein mit einer Aminosäuresequenzhomologie zum C-terminalen Bereich (Aminosäuren 384-641) des Hsp70-Proteins von ≥ 70% vermischt und inkubiert, um eine Aktivierung der NK-Zellen zu bewirken.
Die Inkubation kann bei Raumtemperatur, bevorzugt aber bei physiologischer Temperatur (37°C) auf einem Schüttler (sanftes Schütteln) erfolgen.

In einer bevorzugten Ausführungsform des erfindungsgemäßen Verfahrens umfaßt die Aktivierung eine Stimulation der Proliferation der NK-Zellen und/oder eine Steigerung ihrer Zytotoxizität. Hinsichtlich der bevorzugten Zielzellen für die zytotoxische Aktivität wird auf die vorstehenden Darstellungen verwiesen.

In einer besonders bevorzugten Ausführungsform des erfindungsgemäßen Verfahrens werden als NK-Zellen enthaltende physiologische Zellsuspension periphere, mononukleäre Blutzellen (PBMC) oder eine NK-Zellen enthaltende Fraktion hiervon eingesetzt.

Die NK-Zellen können durch geeignete Verfahren aus den zu behandelnden Patienten oder aus einem gesunden Spender durch Blutabnahme gewonnen werden. Bevorzugt sollen Buffy-Coats (Lymphozytenkonzentrate), die NK-Zellen enthalten, verwendet werden.

Buffy-Coats (Lymphozytenkonzentrate) werden über die Vene aus dem Patienten entnommen und z.B. mit Heparin versetzt, um eine Verklumpung der Zellen zu verhindern. Die mit Heparin versetzten Buffy-Coats werden in einem sterilen Gefäß (zumeist Plastiksäckchen) gesammelt und anschließend zentrifugiert, so daß es zu einer Anreicherung von Blutzellen ( = PBMC, periphere, mononukleäre Blutzellen, z.B. Lymphozyten, Erythrozyten, Granulozyten usw.) kommt. Das Lymphozytenkonzentrat bleibt steril im Gefäß (Plastikbeutel). Im Falle von gesunden Probanden besteht ein Buffy-Coat aus weißen und roten Blutzellen (Lymphozyten, Erythrozyten usw.). Im Falle eines Tumorpatienten besteht der Buffy-Coat nicht nur aus Blutzellen, sondern kann auch Tumorzellen enthalten (bei Leukämien, z.B. leukämische Zellen = Blasten; bei soliden Tumoren, z.B. metastasierte Zellen).

Die Buffy-Coats, die periphere, mononukleäre Blutzellen enthalten, werden in Form einer physiologischen Zellsuspension, bevorzugt versetzt mit Heparin, eingesetzt. Das Heparin verhindert eine Aggregation der Zellen.

In einer weiteren besonders bevorzugten Ausführungsform des erfindungsgemäßen Verfahrens enthält die Zellsuspension weiterhin Hsp70 auf der Zelloberfläche exprimierende menschliche oder tierische Zellen. Eine Stimulation der NK-Zellen durch Hsp70-Protein kann allerdings auch erfolgen, wenn keine Hsp70 auf der Zelloberfläche exprimierende Zielzellen (Tumorzellen, infizierte Zellen) vorhanden sind.

In einer anderen besonders bevorzugten Ausführungsform des Verfahrens werden als menschliche oder tierische Zellen Tumorzellen und Zellen von Patienten mit Infektionserkrankungen eingesetzt.

In einer weiteren besonders bevorzugten Ausführungsform des Verfahrens werden als menschliche oder tierische Zellen Leukämiezellen, Lymphomzellen, metastasierende Zellen solider Tumore und Zellen von Patienten mit viralen, mykotischen oder bakteriellen Infektionserkrankungen eingesetzt.

In einer anderen bevorzugten Ausführungsform des Verfahrens wird die die Zellen und Proteine enthaltende physiologische Zellsuspension mindestens 3 Stunden inkubiert.
Um die zytolytische Wirkung der Natürlichen Killerzellen zu steigern, werden in dieser Ausführungsform vorzugsweise die Zielzellen der Natürlichen Killerzellen zusammen mit den Natürlichen Killerzellen und dem Hsp70 miteinander in Suspension vorzugsweise für den genannten Zeitraum inkubiert. Allerdings sind auch Langzeitinkubationen für mindestens 4 Tage möglich. Dementsprechend wird in einer anderen besonders bevorzugten Ausführungsform des erfindungsgemäßen Verfahrens die Inkubation 4 Tage lang vorgenommen.

In einer weiteren bevorzugten Ausführungsform der erfindungsgemäßen Verwendung oder des erfindungemäßen Verfahrens wird zusätzlich ein Zytokin eingesetzt. Das Zytokin kann dabei getrennt mit den NK-Zellen und/oder den Hitzeschockproteinen, Fragmenten oder Derivaten davon oder zusammen in einer Dosis eingesetzt werden.

In einer besonders bevorzugten Ausführungsform der Verwendung oder des Verfahrens wird als Zytokin ein Interleukin eingesetzt. Auch eine Kombination von Interleukinen kann erfindungsgemäß zusammen mit dem Hsp70-Protein eingesetzt werden, um die Aktivierung der NK-Zellen weiter zu verstärken, z.B. die durch NK-Zellen vermittelte Immunantwort oder die Stimulation der Proliferation der NK-Zellen.

In einer weiteren besonders bevorzugten Ausführungsform der Verwendung oder des Verfahrens der Erfindung wird als Interleukin IL-2, IL-12 und/oder IL-15 eingesetzt.

Durch die Erfindung eröffnet sich die Möglichkeit, nicht nur ex vivo aktivierte NK-Zellen in den Patienten zu reinfundieren, sondern aufgrund der Vermeidung toxischer Stoffe erfindungsgemäß behandelte NK-Zellen, z.B auch in Kombination mit einer Hyperthermiebehandlung, auch in vivo einzusetzen. Diese Ausführungsform der Erfindung hat den weiteren, unschätzbaren und überraschenden Vorteil, daß auch Zielzellen, z.B. Tumorzellen, die den bekannten Therapieverfahren widerstanden, nunmehr durch zytolytische Wirkung von NK-Zellen immunologisch abgetötet werden können. Die Erfindung betrifft somit ferner ein Verfahren zur in vivo-Aktivierung des Immunsystems, wobei man einem Patienten eine pharmazeutisch wirksame Menge von nach dem vorstehend beschriebenen Verfahren aktivierten NK-Zellen verabreicht, gegebenenfalls in Kombination mit oder zeitlich vor einer pharmazeutisch wirksamen Menge eines Hsp70-Proteins, eines C-terminalen Fragmentes davon oder eines Derivats davon oder eines Proteins mit einer Aminosäuresequenzhomologie zum C-terminalen Bereich (Aminosäuren 384-641) des Hsp70-Proteins von ≥ 70%.

Im Falle, daß die Wirkstoffe zusammen verabreicht werden, können die Wirkstoffe in einem Container oder mehreren Containern getrennt formuliert sein, während sie im Falle einer zeitlich getrennten Verarbeitung getrennt formuliert sind.

Sofern die NK-Zellen vor dem Hsp70-Proteinen verabreicht werden, sollte der entsprechende Zeitraum vor der Gabe des Hsp70-Proteins mindestens 3-24 Stunden betragen.

Ein Beispiel für eine erfindungsgemäße Behandlung ist wie folgt:
Buffy-Coat-Zellen (Lymphoztenkonzentrate), bestehend aus peripheren, mononukleären Blutzellen oder Knochenmarkszellen und Tumorzellen aus Tumorpatienten, beispielsweise Leukämiepatienten, werden in einem Behälter, beispielsweise einem Kunststoffbehälter, der steril verschlossen ist, einer Behandlung mit dem Hsp70, Hsp70-verwandten Protein und/oder wirksamen Fragmenten oder Derivaten hiervon, in einem temperaturkontrollierten Wasserbad einer Hitzebehandlung unterzogen. Im Behälter befinden sich sowohl die Tumorzellen als auch die NK-Zellen, die über die vorliegende Behandlung stimuliert werden. Nach Abschluß des erfindungsgemäßen Verfahrens wird die aktivierte NK-Zellen und lysierte Tumorzellen enthaltende Kulturlösung in den Patienten reinfundiert.

Erfindungsgemäß liegen die NK-Zellen gegebenenfalls zusammen mit anderen peripheren, mononukleären Blutzellen, beispielsweise zusammen mit Erythrozyten und Granulozyten und T-Zellen, vor. Bevorzugt werden somit die NK-Zellen nicht alleine verwendet, sondern durch Isolierung von Buffy-Coat-Zellen eine Mischung der peripheren, mononukleären Blutzellen gewonnen. Bei Tumorpatienten enthalten diese Anreicherungen weiterhin Tumorzellen, die durch das erfindungsgemäße Verfahren immunologisch eliminiert werden.

Desweiteren betrifft die Erfindung die Verwendung einer pharmazeutisch wirksamen Menge eines Hsp70-Proteins, eines C-terminalen Fragmentes davon oder eines Derivats davon oder eines Proteins mit einer Aminosäuresequenzhomologie zum C-terminalen Bereich (Aminosäuren 384-641) des Hsp70-Proteins von ≥ 70% zur Herstellung eines Arzneimittels zur in vivo-Aktivierung von NK-Zellen.

Weiterhin betrifft die Erfindung die Verwendung einer pharmazeutisch wirksamen Menge von nach dem vorstehend beschriebenen, erfindungsgemäßen Verfahren aktivierten NK-Zellen und/oder eines Hsp70-Proteins, eines C-terminalen Fragmentes davon oder eines Derivats davon oder eines Proteins mit einer Aminosäuresequenzhomologie zum C-terminalen Bereich (Aminosäuren 384-641) des Hsp70-Proteins von ≥ 70% zur Herstellung eines Arzneimittels zur Behandlung von Tumoren, Krebserkrankungen und/oder Infektionskrankheiten.

In einer bevorzugten Ausführungsform der Verwendung der Erfindung ist der Tumor ein solider Tumor oder eine Metastase. Die Behandlungsstrategie zielt insbesondere auf die Elimination von Einzelzell-Metastasen ab, die durch die erfindungsgemäße Verwendung immunologisch eliminiert werden können. Eine verstärkte Aktivierung Hsp70-spezifischer NK-Zellen kann durch eine Zugabe von Interleukin-2 in einer niedrigen Dosis, beispielsweise 100 IE, erreicht werden. Das Interleukin-2 kann beispielsweise zusammen mit dem Hsp70 in den sterilen Behälter, beispielsweise einen Kunststoffbehälter, eingeführt werden.

In einer weiteren bevorzugten Ausführungsform der Erfindung ist die Krebserkrankung Leukämie oder ein Lymphom.

In einer anderen bevorzugten Ausführungsform der Erfindung ist die Infektionskrankheit viralen, mykotischen oder bakteriellen Ursprungs.

Die Erfindung betrifft ferner ein Arzneimittel, einen medizinischen Hilfsstoff, oder ein Medizinprodukt, das ein C-terminales Fragment von Hsp70-Protein oder ein Derivat davon oder von nach dem erfindungsgemäßen Verfahren aktivierten NK-Zellen in einer therapeutisch wirksamen Menge sowie gegebenenfalls übliche Träger- und/oder Hilfsstoffe enthält. Dem Arzneimittel ist gegebenenfalls ferner ein wie oben definiertes Zytokin zugesetzt.

In einer bevorzugten Ausführungsform des Arzneimittels, medizinischen Hilfsstoffs oder Medizinproduktes liegt das Protein in einer Konzentration von mindestens 1 µg/ml, bevorzugt bis zu 1000 µg/ml, vorzugsweise 1 x 10⁶ bis 5 x 10⁸ NK-Zellen, wobei eine Menge von 10 µg bis 600 µg/ml bevorzugt ist.

Beispiele für geeignete pharmazeutisch verträgliche Träger sind dem Fachmann geläufig und umfassen beispielsweise phosphatgepufferte Salzlösungen, Wasser, Emulsionen, wie z. B. Öl/Wasser-Emulsionen, sterile Lösungen, etc. Die pharmazeutischen Zusammensetzungen (Arzneimittel), die derartige Träger enthalten, können nach gängigen Verfahren formuliert werden. Die pharmazeutischen Zusammensetzungen können dem betroffenen Individuum in einer geeigneten Dosis verabreicht werden. Arten der Verabreichung sind beispielsweise intravenös, intraperitoneal, subkutan, intramuskulär, topisch oder intradermal. Die Dosierung hängt dabei von vielen Faktoren ab, z. B. von der Größe, dem Geschlecht, dem Gewicht, dem Alter des Patienten, sowie der Art der speziell verabreichten Verbindung, der Art der Administration etc. Im allgemeinen liegt die monatlich verabreichte Dosis bei 10 bis 1000 µg. Im Zusammenhang mit der intravenösen Injektion von erfindungsgemäßen Substanzen sind Dosierungen von 10 bis 1000 µg gängig. Die Zusammensetzungen können lokal oder systemisch verabreicht werden. Im allgemeinen wird die Verabreichung parenteral erfolgen. So werden die erfindungsgemäß mit Hsp70-Protein behandelten NK-Zellen bevorzugt intravenös injiziert. Es kann auch eine Injektion direkt in den Tumor erfolgen, wobei eine wirksame Menge der NK-Zellen injiziert wird. Selbstverständlich sind auch andere, an sich bekannte Applikationsformen möglich.

Das Hsp70-Protein selbst kann beispielsweise zusammen mit Zytokinen appliziert werden. Ein Beispiel für eine Applikation ist die Injektion des Hsp70-Proteins, z.B. zusammen mit Zytokinen intravenös, intramuskulös, subkutan, intraperitoneal oder auch in die Fußsohle.

In einer anderen bevorzugten Ausführungsform der Verwendung oder des Verfahrens oder des Arzneimittels, des Medizinprodukts oder des medizinischen Hilfsmittels der Erfindung ist das Hsp70-Protein ein humanes Protein. Beispielsweise ist das erfindungsgemäße Protein humanen Ursprungs (bei der Isolierung aus Zellextrakten) bzw. weist die Aminosäuresequenz des menschlichen Hsp70-Proteins auf (z. B. nach rekombinanter Herstellung). Es können aber auch tierische Hsp70-Proteine eingesetzt werden.

Das erfindungsgemäß eingesetzte Hsp70-Protein oder die Fragmente können sowohl rekombinant hergestellt werden, aus Zellextrakten isoliert werden oder über chemische Synthese hergestellt werden.
Bevorzugt ist, daß das Hsp70-Protein oder dessen Fragment oder Derivat ein rekombinantes Protein ist. Derartige rekombinante Proteine können nach Standardverfahren hergestellt werden.

Diese Standardverfahren sind dem Fachmann bekannt (Sambrook et al., loc. cit. und Ausubel, "Current Protocols in Molecular Biology", Green Publishing Associates and Wiley Interscience, N.Y. (1989)). Zur rekombinanten Herstellung der Proteine werden Nukleinsäuremoleküle eingesetzt, die das erfindungsgemäße Hsp70-Protein oder Fragmente hiervon kodieren. Dies können verschiedene Nukleinsäuremoleküle sein, insbesondere DNA oder RNA Moleküle, beispielsweise cDNA, genomische DNA, mRNA etc. Diese Nukleinsäuremoleküle können natürlich vorkommende Moleküle sein und/oder durch genetische oder chemische Syntheseverfahren hergestellte Moleküle. Um rekombinante Proteine herzustellen, verwendet der Fachmann Vektoren, insbesondere Plasmide, Cosmide, Viren, Bakteriophagen und andere in der Gentechnik gängige Vektoren (Sambrook et al., loc. cit.). Die in den Vektoren enthaltenen Nukleinsäuremoleküle können verknüpft sein mit regulatorischen Elementen, die die Expression in prokaryontischen oder eukaryontischen Zellen gewährleistet. Hierbei kann der Begriff "Expression" Transkription als auch Transkription und Translation bedeuten. Regulatorische Elemente umfassen dabei insbesondere Promotoren. Für die Expression eines Nukleinsäuremoleküls in prokaryontischen Zellen stehen eine Reihe von Promotoren zur Verfügung, z. B. der E. coli lac- oder trp-Promotor, der P_{R}- oder P_{L}-Promotor des Lambda-Phagen, lacI, lacZ, T3, T7, gpt, etc. Eukaryontische Promotoren sind beispielsweise der CMV immediate early-Promotor, der HSV-Promotor, der Thymidinkinase-Promotor, der SV40-Promotor, LTRs von Retroviren und der Maus Metallothionin I-Promotor. Es ist bereits eine Vielzahl von Expressionvektoren für die Expression in prokaryontischen oder eukaryontischen Zellen beschrieben, z. B. für Eukaryonten pKK223-3 (Pharmacia Fine Chemicals, Uppsala, Schweden) oder GEM1 (Promega Biotec, Madison, WI, USA), pSV2CAT, pOG44 und für Prokaryonten pQE70, pQE60, pBluescript SK, etc. Neben Promotoren können diese Vektoren auch Elemente zur weiteren Steigerung der Transkription enthalten, wie z. B. sogenannte Transkriptions-Enhancer. Beispiele dafür sind der SV40-Enhancer, der Polyoma-Enhancer, der Cytomegalovirus early promoter-Enhancer und Adenovirus-Enhancer. Die rekombinanten Proteine können daher in verschiedenen prokaryontischen oder eukaryontischen Wirtszellen, beispielsweise unter Einsatz der oben beschriebenen Vektoren, exprimiert werden. Beispiele für solche Wirtszellen sind bakterielle Zellen (wie z. B. E. coli, Streptomyces, Bacillus, Salmonella typhimurium), Pilzzellen (wie beispielsweise Hefezellen, insbesondere Saccharomyces cerevisiae), Insektenzellen (wie. z. B. Drosophila- oder SF9-Zellen), tierische Zellen (wie z. B. CHO oder COS-Zellen) oder auch Pflanzenzellen, etc. Solche Wirtszellen werden unter Bedingungen kultiviert, die die Expression des rekombinanten Proteins erlauben, welches anschließend aus den Zellen und/oder aus dem Kulturmedium gewonnen werden kann. Verfahren zur Expression von Fremdprotein in verschiedenen Arten von Wirtszellen sowie zur Gewinnung des produzierten Proteins sind dem Fachmann geläufig.

In einer anderen bevorzugten Ausführungsform der Verwendung oder des Verfahrens oder des Arzneimittels, Medizinprodukts oder medizinischen Hilfsstoffs der Erfindung umfaßt das Hsp70-Protein das C-terminale Fragment die Aminosäuren 384 bis 561 von menschlichem Hsp70 oder den entsprechenden Bereich aus einem anderem Hsp70, das die erfindungsgemäßen Wirkungen aufweist oder umfaßt das C-terminale Fragment die Aminosäuren 454 bis 460 von menschlichem Hsp70. Erfindungsgemäß konnte überraschenderweise gezeigt werden, daß Fragmente, welche diese minimale Sequenz von 7 Aminosäuren (NLLGRFE) haben, durch Antikörper gehemmt werden, wodurch NK-Aktivierung unterbunden bzw. getrennt wurde. Die Experimente wurden entsprechend Multhoff et al., J. Immunol. 158 (1997), 4341-4350 durchgeführt. Es wurde der von Amersham erhältliche Antikörper RPN 1197 eingesetzt. Die 7 Aminosäuren können dabei von natürlicherweise flankierenden Hsp70-Sequenzen oder durch andere Aminosäuren flankiert sein. Bevorzugt ist, daß die 7 Aminosäuren in ihrem natürlichen Kontext verbleiben. Sofern andere flankierende Aminosäuren eingesetzt werden, wird bevorzugt der dreidimensionale Kontext, in dem die genannten 7 Aminosäuren natürlicherweise stehen beibehalten. Innerhalb der 7 Aminosäuren können weitere Aminosäureaustausche erfolgen, sofern die Homologie von mindestens 70% beibehalten wird. Allerdings umfassen diese Austausche nicht einen Austausch von Arginin in Position 458 durch Lysin. Dieser Austausch führt zu einer Konformationsänderung. Entsprechend sind Austausche, die in den Bereich der 7 Aminosäuren zu einer Konformationsänderung führen nur dann von der Erfindung umfaßt, wenn sie die gewünschten Aktivierungseigenschaften aufweisen.

Die Erfindung betrifft ferner die Verwendung der nach einem Verfahren nach einem oder mehreren der vorhergehenden Ausführungsformen behandelten NK-Zellen zur Therapie von Tumorerkrankungen, und/oder Infektionserkrankungen.

In einer bevorzugten Ausführungsform der erfindungsgemäßen Verwendung erfolgt die Therapie durch Reinfundierung der behandelten NK-Zellen.

Die vorstehend beschriebenen in vivo Verfahren können auch als Behandlungsverfahren zur Behandlung der beschriebenen Indikatoren eingesetzt werden.

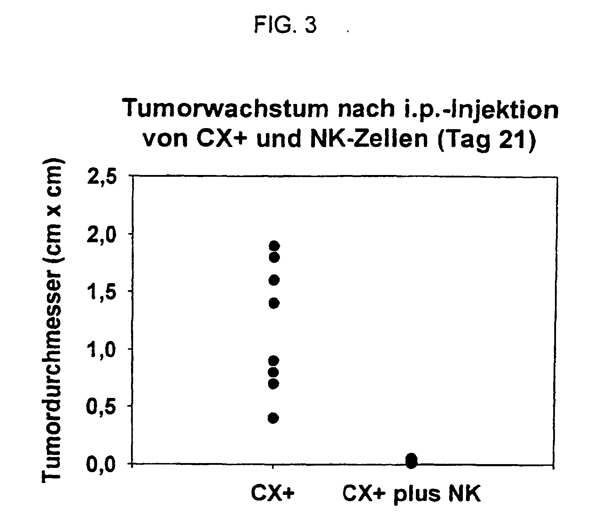

Die Figuren zeigen:

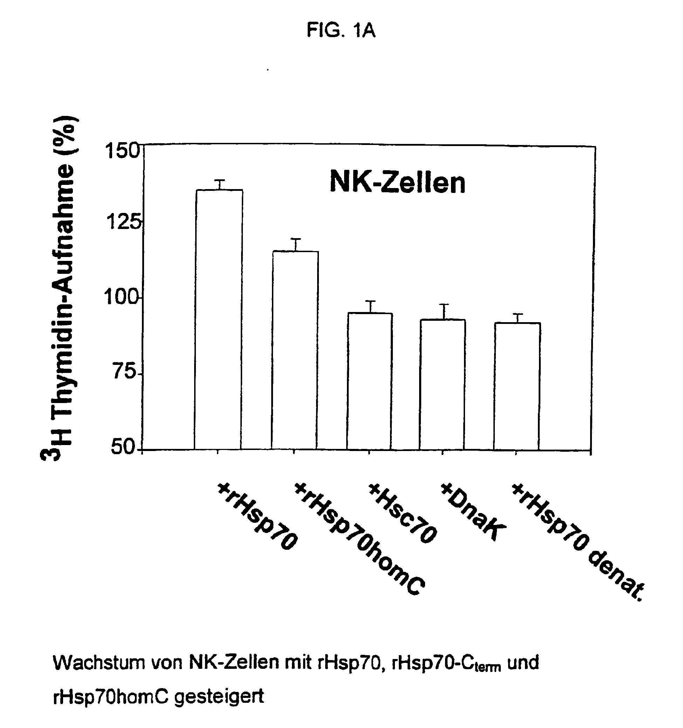

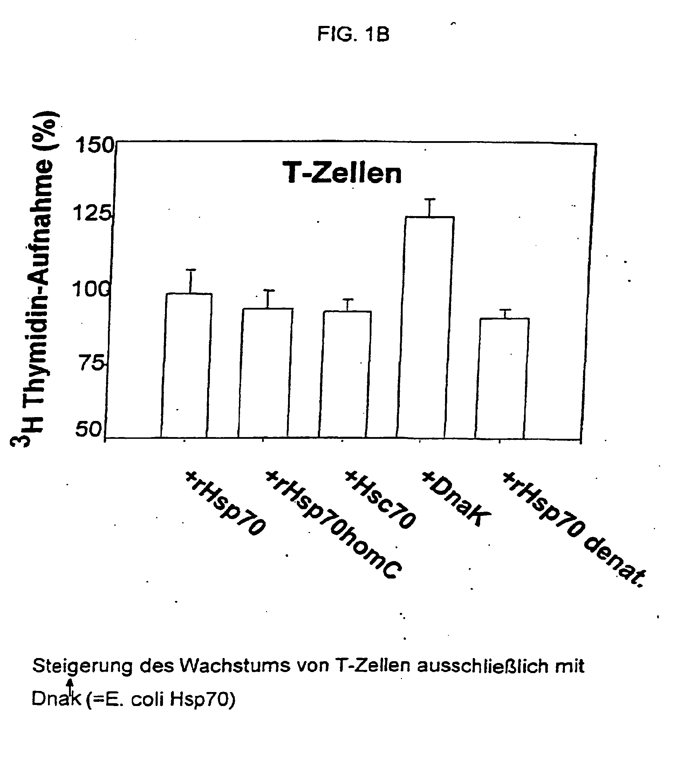

- FIGUR 1:: Vergleich der proliferierenden Aktivität getrennter NK-(A)- und T(B)-Zellen, die entweder mit IL-2 (100 IU/ml)-Medium oder mit anderen rekombinanten Hsp70-Proteinen (rHsp70, rHsp70-Cₜₑᵣₘ. (Aminosäuren 384-561), rHsp70homC, DnaK, Hsc70 und hitzedenaturiertem rHsp70), die in IL-2-Medium (100 IU/ml) suspendiert sind, je mit einer Konzentration von 10 µg/ml, stimuliert wurden. Die phänotypische Charakterisierung der NK-Zellen ist wie folgt:
CD3 : < 5%; CD16/CD56 : 46-87%; CD94 : 60-70%; p58.1 und p58.2 : < 5% und T-Zellen: CD3 : 85-92%; CD16/CD56 : 5-10%; CD94 : < 29%; p58.1 und p58.2: nicht getestet; p70: nicht getestet, durchflußzytometrisch bestimmt. Die Proliferation der Zellen wurde nach 48 Stunden und nach Inkubation mit ³H-Thymidin (1 µCi/ml) bei 37°C für 18 Stunden bestimmt. Der relative Prozentsatz der ³H-Thymidin-Aufnahme in NK-(A) und T(B)-Zellen wurde mit den Wirkungen von IL-2 allein (100%) verglichen. Die Werte zeigen die Mittelwerte von vier bis sieben unabhängigen Experimenten ± Standardabweichung.

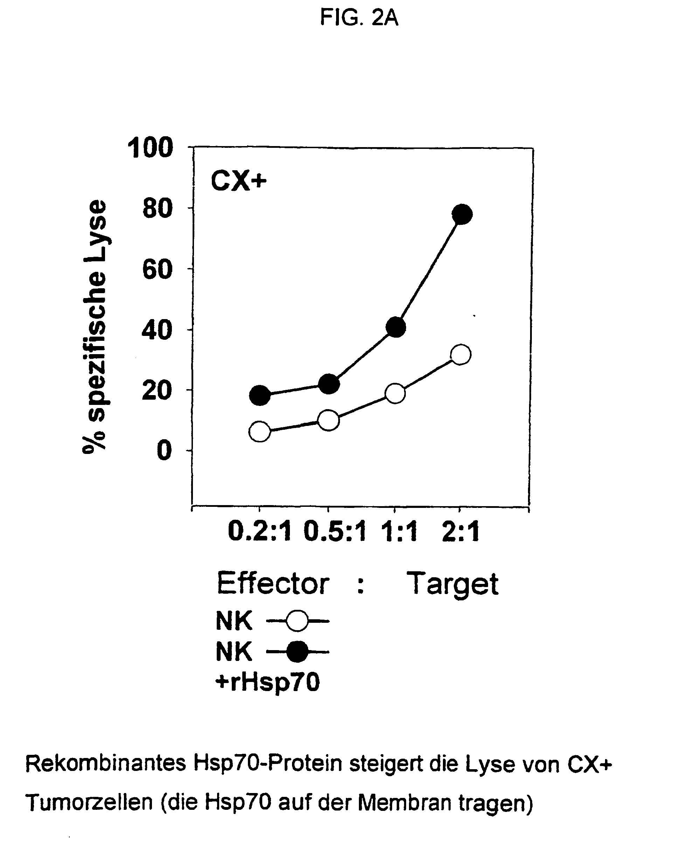

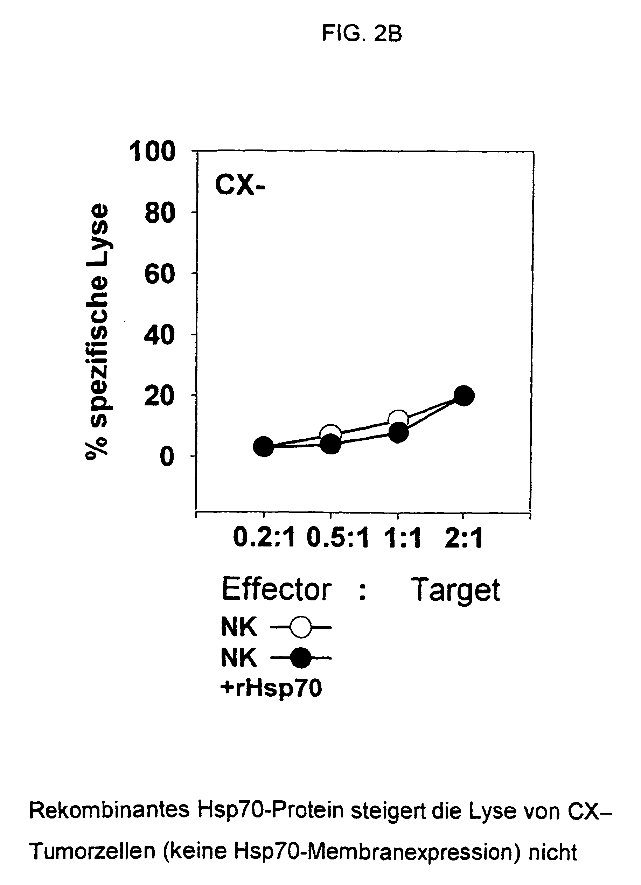

- FIGUR 2:: Vergleich der zytotoxischen Aktivität hochgereinigter NK-Zellen (CD3 : < 2%; CD16/CD56 : 75-80%; CD94 : 65-87%; p58.1 und p58.2 : 20-30%; p70 : < 10%), die entweder unbehandelt blieben (durchgezogene Linien, leere Symbole) oder nach einer Vorinkubation der NK-Zellen mit rHsp70 (A)-Protein (je 5 µg/ml für 4 Tage; durchgezogene Linien, ausgefüllte Symbole) vorinkubiert wurden, gegenüber ⁵¹Cr-markierten Tumortargetzellen CX+ (A) und CX- (B), die sich aufgrund ihrer Fähigkeit, Hsp70 auf ihrer Plasmamembran zu exprimieren, unterscheiden. Die Ergebnisse sind als Prozentsatz der spezifischen Lyse bei verschiedenen E : T-Verhältnissen von 0,2 : 1 bis 2 : 1 ausgedrückt. Jeder Punkt stellt den Mittelwert zumindest dreier unabhängiger Experimente ± Standardabweichung dar. Der Prozentsatz an spontaner Freisetzung für jede Tumortargetzellinie war immer unter 15%.
- FIGUR 3:: Tumorwachstum von Hsp70 tragenden CX+ Zellen in immundefizienten Mäusen. Nach i. p. -Injektion von NK-Zellen wird das Tumorwachstum vollständig inhibiert (bei i.p.-Injektion der Tumorzellen). Die Tumorgröße wurde in cm² angegeben.
Tumorwachstum nach i.p.-Injektion von CX+ und NK-Zellen am Tag 21.

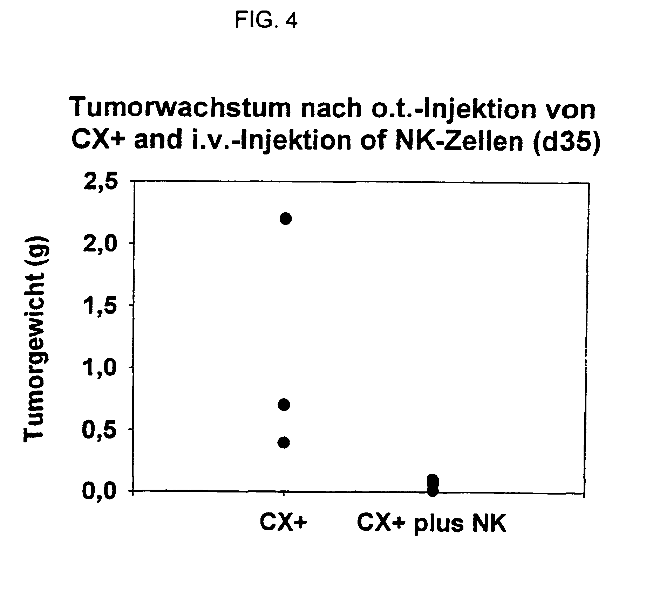

- FIGUR 4:: Tumorwachstum von Hsp70 tragenden CX+ Zellen in immundefizienten Mäusen. Nach i. v.-Injektion von NK-Zellen wird das Tumorwachstum vollständig inhibiert. Das Tumorwachstum wurde in Gramm gemessen.
Tumorwachstum nach o.t.-Injektion von CX+ und i.v. -Injektion von NK-Zellen am Tag 35. Die NK-Zellen verhindern das Tumorwachstum von Hsp70-tragenden CX+ Zellen nach 3 bzw. 5 Wochen nach Injektion und (vgl. Figur 3). Sowohl eine intraperitoneale als auch eine intravenöse Applikation der NK-Zellen führt zu vergleichbaren Ergebnissen.
- FIGUR 5:: Intaktes Hsp70-Protein mit Darstellung des C-terminalen Bereichs

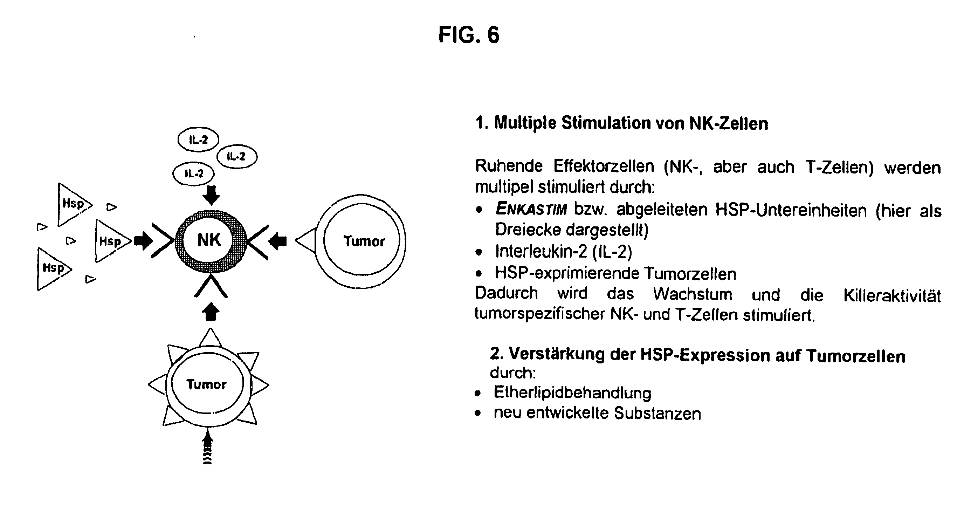

- FIGUR 6:: Darstellung des Einflusses von Hsp70 und/oder Zytokinen auf die durch NK-Zellen vermittelte Immunantwort. Die Zytokinzugabe bewirkt auch eine Stimulation von T-Zellen.

Die Beispiele erläutern die Erfindung.

### Beispiel 1: Gesteigerte Proliferation von NK-Zellen nach Gabe von Hsp70

Die Proliferation gereinigter NK- und T-Zellen, die mit den Hsp70-Proteinen rHsp70, DnaK, Hsc70, rHsp70-C term. und rHsp70homC (Aminosäuren 384-561) stimuliert worden waren, wurde in einem ³H-Thymidin-Aufnahme-Standardtest bestimmt (Testbedingungen vgl. später). Dazu wurden zunächst periphere Blutlymphozyten aus freiwilligen, menschlichen Spendern in nicht-adhärente CD3+ T-Zell und transient (12-24 Stunden) adhärente CD3-(CD16+/CD56+) NK-Zell-Subpopulationen in einem Mehrschrittverfahren und nachfolgender 12-stündiger Inkubation in einem IL-2-enthaltendem Medium (vgl. 3) getrennt. Die Zellen wurden getrennt in rIL-2 (100 IE, Chiron, Frankfurt, Deutschland), enthaltend ein RPMI 1640 (Life Technologies, Eggenstein, Deutschland)-Medium für 3-4 Tage lang kultiviert. Die Proliferation wurde als H-3 Aufnahme gemessen.

Panning-Experimente wurden unter Verwendung von humanem, rekombinanten Hsp70 (rHsp70, SPP-755, StressGen Biotechnologies, Victoria, Canada) und DnaK (Hsp70-Homolog, erhalten aus E. coli, SPP-630, StressGen) durchgeführt. Die Proteine wurden in PBS zu einer Stammkonzentration von 1 µg/ml verdünnt und in Aliquots bei -80°C tiefgefroren. T-25-Kulturflaschen wurden mit rHsp70 bzw. DnaK-Protein (10 µg/ml), verdünnt in 3 ml eiskaltem Carbonatpuffer, pH 9,5 12 Stunden lang inkubiert. Nach Abblockung nicht-spezifischer Bindungsstellen mit PBS/5% FKS wurde eine Mischung aus T- und NK-Zellen suspendiert in PBS/1% FKS in einem Verhältnis von 1 : 2 und 2 : 1 in den Kulturflaschen 1 Stunde lang bei Raumtemperatur inkubiert. Nicht-adhärente Zellen wurden aus der Überstandsfraktion nach Inkubation erhalten. Adhärente Zellen wurden durch sequentielle Waschschritte unter Verwendung von eiskalter PBS/10% FKS-Lösung erhalten. Um leicht-adhärente Zellen zu entfernen, wurde ein einzelner, milder Waschschritt verwendet, während stark-adhärente Zellen durch zusätzliche, stringente Waschschritte erhalten wurden. Die bei jedem Schritt erhaltenen Zellpopulationen wurden getrennt gezählt und durchflußzytometrisch, phänotypisch charakterisiert.

Die Durchflußzytometrie wurde wie in (4) beschrieben auf einem FACScan-Instrument (Becton Dickinson, Heidelberg, Deutschland) durchgeführt. Der Prozentsatz an positiv-gefärbten Zellen wurde definiert als Differenz zwischen der Zahl spezifisch-gefärbter, vitaler (propidiumiodid-negativer) Zellen minus der Anzahl an Zellen, die mit dem Isotyp-entsprechenden Kontrollantikörpern gefärbt waren. Die nachfolgenden Antikörper wurden zur phänotypischen Kennzeichnung der Effektorzellen verwendet:
Dem Isotyp-entsprechender Kontrollantikörper (Dianova, Hamburg, Deutschland; Becton Dickinson, Heidelberg, Deutschland), CD16 (Dianova, Hamburg, Deutschland), CD3 (Dianova, Hamburg, Deutschland).

Die Proliferationsfähigkeit von T- oder NK-Zellen gegen unterschiedliche Hsp70-Proteine wurde in einem ³H-Thymidin-Aufnahme-Standardtest bestimmt. Lebensfähige Zellen (5 x 10⁴ Zellen/100 µl) wurden in eine 96 Vertiefungen-enthaltende Mikrotiterplatte mit einem flachen Boden (Greiner, Nürtingen, Deutschland) eingesät, wobei ein supplementiertes RPMI 1640-Medium mit 100 IE IL-2 und verschiedenen, rekombinanten Hsp70-Proteinen (rHsp70, DnaK, Hsc70, die konstitutive Form von Hsp70, gereinigt aus Rinderhirn, SPP-750; StressGen; rHsp70-C term., sie rek.
C-terminale Peptidbindungsdomäne von Hsp70 (Aminosäuren 384 - 561), rHsp70homC, die rekombinante C-terminale peptidbindungsdomäne von Hsp70hom (Hsp70hom ist ein Testis spezifisches Mitglied der Hsp70 Familie, das eine starke Homologie (94%) zu Hsp70 aufweist), Aminosäuren 384-561) eingesetzt wurde. Durch Test verschiedener Konzentrationen der Hsp70-Proteine (1 - 200 µg/ml) konnte herausgefunden werden, daß eine Endkonzentration von 100 µg/ml zur Stimulation optimal war. Als weitere interne Kontrolle wurde die proliferierende Aktivität gegen IL-2 (100 IE) parallel bestimmt. Nach einer Inkubationszeit von 24 Stunden oder 48 Stunden wurden die Zellen mit ³H-Thymidin (1 µCi/Vertiefung) markiert, und die Gesamtaufnahme wurde nach 18-stündiger Inkubation bei 37°C in einem Flüssigszintillationszählgerät (Beckmann Instruments, München, Deutschland) bestimmt. Als interne Kontrolle wurde weiterhin die Proliferationskapazität von aus dem gleichen Spender stammenden T-Lymphozyten bestimmt. Dosiseskalierungsuntersuchungen unter Verwendung verschiedener Hsp70-Proteine/Fragmente in Konzentrationen von 1-200 µg/ml zeigten, daß eine maximale Stimulierung der Proliferationskapazität mit 100 µg/ml Hsp70-Protein erreichbar war. Die Proliferationsaktivität isolierter NK- und T-Zellen wurde nach in vitro-Stimulation mit rHsp70, DnaK, Hsc70, rHsp70-C term. bzw. rHsp70homC (Aminosäuren 384-562) getestet. Wie in der Figur 1A gezeigt, wurde die NK-Zellproliferation durch rHsp70 signifikant stimuliert. Die Stimulation durch den carboxy-terminalen Bereich von Hsp und durch rHsp70homC, das in der C-terminalen Domäne mit den Aminosäuren 384-561 zu 94% mit Hsp70 identisch ist, ist ebenfalls möglich. Im Gegensatz dazu stimulierten DnaK und Hsc70 die Proliferation von NK-Zellen nicht. Hitzedenaturiertes rHsp70 verlor die stimulatorischen Eigenschaften für die Proliferation von NK-Zellen vollständig.

Weiterhin konnte gezeigt werden, daß die Proliferation von CD3positiven T-Lymphyten durch DnaK stimulierbar war, während rHsp70, Hsc70 und rHsp70homC und hitzedenaturiertes rHsp70 keine Wirkung auf die Proliferationsfähigkeit von T-Zellen zeigten (Figur 1B).

Zusammenfassend konnte somit eine Proliferation von NK-Zellen durch rekombinantes humanes Hsp70-Protein durch den C-terminalen Bereich von Hsp70 (384 - 561) und rHsp70homC, einem zum Hsp70 homologen Protein, induziert werden, während eine Proliferation der T-Zellen selektiv durch bakterielles Hsp70 (E. coli DnaK) stimulierbar war.

### Beispiel 2: Steigerung der zytolytischen Aktivität von NK-Zellen nach Gabe von Hsp70

Eine funktionelle Analyse der NK-Zellen unter Verwendung von Hsp70-exprimierenden (CX+) und Hsp70-nicht-exprimierenden. (CX-)-Tumorzellen zeigte, daß die Plasmamembranexpression von Hsp70 mit einer erhöhten Sensitivität für die durch NK-Zellen vermittelte Lysis korrelierte. Diese durch NK-Zellen vermittelte Lysis von Tumorzellen kann durch Vorinkubation der Tumorzellinien mit monoklonalen Antikörpern, die gegen den carboxy-terminalen Bereich (Aminosäuren 504-617) von Hsp70 gerichtet sind und mit dem Antikörper RPN1197 (1, 4), blockiert werden. Nachfolgend wurde der Einfluß von rekombinantem Hsp70-Protein (rHsp70) auf die zytolytische Aktivität von NK-Zellen für die autologen, Hsp70-exprimierenden (CX+)- und Hsp70-nichtexprimierenden (CX-)-Tumorzellen analysiert. Die Ergebnisse der Zytotoxizitätstests unter Verwendung hochgereinigter NK-Zellen, die mit Hsp70-Protein in einer Konzentration von 5 µg/ml für 4 Tage vorinkubiert worden waren, sind in der Figur 2A und B zusammengefaßt. Die Versuchsansordnung war dabei wie folgt: Zur Stimulation der zytotoxischen Aktiviatät wurden NK-Zellen mit 10 µg/ml rHsp70 inkubiert. Die Stimulation wurde in 4-tägigem Abstand wiederholt.

Die humanen, autologen Kolonkarzinom-Subzellinien CX+ und CX-, die sich in ihrer Hsp70-Expression auf der Plasmamembran unterscheiden (Multhoff et al., J. Immunol. 158 (1997), 4341), wurden in RPMI 1640-Medium, supplementiert mit 10% FKS (Life Technologies), 6 mM L-Glutamin und Antibiotika (100 IE/ml Penicillin und 100 µg/ml Streptomycin; Life Technologies) kultiviert. Exponentiell wachsende Tumorzellen wurden als Targetzellen verwendet, und gereinigte CD3- NK-Zellen wurden, nach Zellsortierung unter Verwendung von eines FACStar^{plus}-Instruments (Becton Dickinson, Heidelberg, Deutschland), als Effektorzellen eingesetzt. Die durch NK-Zellen vermittelte Zytotoxizität wurde unter Verwendung eines 4-stündigen ⁵¹Cr-Radioisotopentests (Multhoff et al., J. Immunol. 158 (1997), 4341) bestimmt. Der Prozentsatz der spezifischen Lyse wurde wie folgt berechnet: [(experimentelle Freisetzung - spontane Freisetzung)/(maximale Freisetzung - spontane Freisetzung)] x 100. Die spontane Freisetzung von Cr-51 betrug in allen Versuchen unter 15%.

Überraschenderweise konnte gezeigt werden, daß bei einer Inkubation der NK-Zellen mit rHsp70-Protein für mindestens 4 Tage sowohl die Proliferation als auch die zytolytische Aktivität von NK-Zellen gegenüber Hsp70-exprimierenden Tumorzellen (CX+) stimuliert wurde. Im Gegensatz dazu verloren NK-Zellen aus dem gleichen Spender, die nicht mit rHsp70 behandelt wurden, diese Reaktivität nach 10 Tagen. Die lytische Aktivität von nicht mit rHsp70 stimulierten NK-Zellen war im Vergleich zu mit rHsp70 behandelten und stimulierten NK-Zellen geringer, und es konnte dann kein signifikanter Unterschied in der Lyse Hsp70exprimierender und nicht-exprimierender Tumorzellen festgestellt werden.

Die vorliegenden Untersuchungen zeigen, daß der carboxy-terminale Teil von Hsp70 (Aminosäuren 384 - 641), für die Stimulierung der zytolytischen und proliferativen Funktion von NK-Zellen verantwortlich ist. Erfindungsgemäß konnte somit gezeigt werden, daß insbesondere der carboxy-terminale Teil des Hsp70-Proteins als stimulierendes Signal für NK-Zellen wirkt, die spezifisch Hsp70-exprimierende Tumorzellen in vitro angreifen.

### Beispiel 3: Anti-tumorale Wirkung mit Hsp70 stimulierter NK-Zellen

Für die Untersuchung der in vivo Relevanz von NK-Zellen gegenüber Hsp70-exprimierenden Tumorzellen wurden Untersuchungen an immundefizienten SCID/beige Mäusen durchgeführt. Dabei wurden zunächst unterschiedliche Mengen an Tumorzellen (CX+ oder CX-Zellen) in SCID/beige Mäuse injiziert. Eine Menge von 2,5 Mio Zellen erwies sich als optimale Tumorzellmenge zur Induktion von Tumorwachstum innerhalb eines Zeitraums von 3 bis 5 Wochen. Als Injektionsmethode wurde eine i.p. (intraperitoneale) oder o.t. (orthotope, d. h. hier in die Darmwand) Injektion von Kolonkarzinomzellen CX+ oder CX- in die Darmwand gewählt. Die NK-Zellen wurden nach Stimulation entweder i.p. oder i.v. (intravenös) appliziert. Wie in Figur 3 dargestellt, konnte in allen Tieren ein Tumorwachstum sowohl nach i.p.- als auch nach o.t.-Injektion erzielt werden. Im Gegensatz zur i.p.-Injektion konnte nach o.t.-Injektion neben dem Wachstum eines Primärtumors auch eine Metastasierung der CX+ Zellen vor allem in Milz und Lunge beobachtet werden (3 von 3 Mäusen nach o.t.-Injektion).

Eine Injektion von humanen NK-Zellen (i.p., aber auch i.v.) selbst vier Tage nach der Injektion von Tumorzellen führt zu einer vollständigen Inhibition des Tumorwachstums. Interessanterweise konnte durch NK-Zellen nicht nur das Wachstum von Primärtumoren (im i.p. Raum oder am Darm) inhibiert werden, sondern auch die Metastasierung der Tumore.

Diese Befunde machen deutlich, daß eine Immunrekonstitution von SCID/beige Mäusen mit voraktivierten, humanen NK-Zellen nicht nur in vitro, sondern auch in vivo (im Tier) zu einer Lyse von Tumorzellen führt. Interessanterweise kann auch die Metastasierung durch humane NK-Zellen unterdrückt werden.

## Patentansprüche

1. Verwendung eines Hsp70-Proteins, das nicht mit Peptiden aus Tumorzellen komplexiert ist, eines C-terminalen Fragmentes davon oder eines Derivats davon oder eines Proteins mit einer Aminosäuresequenzhomologie zum Bereich von Aminosäuren 384-641 des Hsp70-Proteins von > 70% zur Herstellung eines Arzneimittels, Medizinprodukts oder medizinischen Hilfsstoffs zur Behandlung von Tumoren, Krebserkrankungen oder Infektionskrankheiten durch Aktivierung von NK-Zellen.

2. Verwendung eines Hsp70-Proteins, das nicht mit Peptiden aus Tumorzellen komplexiert ist, eines C-terminalen Fragmentes davon oder eines Derivats davon oder eines Proteins mit einer Aminosäuresequenzhomologie zum Bereich von Aminosäuren 384-641 des Hsp70-Proteins von > 70% zur in vitro oder ex vivo Aktivierung von NK-Zellen.

3. Verwendung nach Anspruch 1 oder 2, wobei die Aktivierung die Induktion einer durch NK-Zellen vermittelten Immunantwort umfasst.

4. Verwendung nach einem der Ansprüche 1 bis 3, wobei die Aktivierung eine Stimulation der Proliferation von NK-Zellen und/oder eine Steigerung der zytolytischen Aktivität von NK-Zellen einschließt.

5. Verwendung nach Anspruch 4, wobei die zytolytische Aktivität gegenüber Tumorzellen oder infizierten Zellen von Patienten mit Infektionserkrankungen gesteigert ist.

6. Verwendung nach Anspruch 5, wobei die zytolytische Aktivität gegenüber Leukämiezellen, Lymphomzellen, Tumorzellen, metastasierenden Zellen solider Tumore oder mit Viren, Bakterien und/oder Pilzen infizierten Zellen von Patienten gesteigert ist.

7. Verfahren zur ex vivo oder in vitro Aktivierung von NK-Zellen, wobei man eine NK-Zellen enthaltende physiologische Zellsuspension mit einem wie in Anspruch 1 definierten Protein, Fragment oder Derivat vermischt und inkubiert, um eine Aktivierung der NK-Zellen zu bewirken.

8. Verfahren nach Anspruch 7, wobei die Aktivierung eine Stimulation der Proliferation der NK-Zellen und/oder eine Steigerung ihrer Zytotoxizität umfasst.

9. Verfahren nach Anspruch 7 oder 8, wobei als NK-Zellen enthaltende physiologische Zellsuspension periphere, mononukleäre Blutzellen oder eine NK-Zellen enthaltende Fraktion hiervon eingesetzt werden.

10. Verfahren nach einem der Ansprüche 7 bis 9, wobei die Zellsuspension weiterhin Hsp70 auf der Zelloberfläche exprimierende menschliche oder tierische Zellen enthält.

11. Verfahren nach Anspruch 10, wobei als menschliche oder tierische Zellen Tumorzellen oder infizierten Zellen von Patienten mit Infektionserkrankungen eingesetzt werden.

12. Verfahren nach Anspruch 11, wobei als menschliche oder tierische Zellen Leukämiezellen, Lymphomzellen, Tumorzellen, metastasierende Zellen solider Tumore oder mit Viren, Bakterien und/oder Pilzen infizierte Zellen von Patienten eingesetzt werden.

13. Verfahren nach einem der Ansprüche 7 bis 12, wobei die die Zellen und Proteine enthaltende physiologische Zellsuspension mindestens 3 Stunden inkubiert wird.

14. Verfahren nach Anspruch 13, wobei die Inkubation 4 Tage lang vorgenommen wird.

15. Verwendung nach einem der Ansprüche 1 bis 6 oder Verfahren nach einem der Ansprüche 7 bis 14, wobei zusätzlich ein Zytokin eingesetzt wird.

16. Verwendung oder Verfahren nach Anspruch 15, wobei als Zytokin ein Interleukin eingesetzt wird.

17. Verwendung oder Verfahren nach Anspruch 16, wobei als Interleukin IL-2, IL-12 oder IL-15 eingesetzt wird.

18. Verwendung einer pharmazeutisch wirksamen Menge von nach dem Verfahren nach einem der Ansprüche 7 bis 17 aktivierten NK-Zellen zur Herstellung eines Arzneimittels zur in vivo-Aktivierung des Immunsystems, wobei das Arzneimittel gegebenenfalls in Kombination mit oder zeitlich vor einer pharmazeutisch wirksamen Menge eines wie in Anspruch 1 definierten Proteins, Fragmentes oder Derivats verabreicht wird.

19. Verwendung einer pharmazeutisch wirksamen Menge von nach dem Verfahren nach einem der Ansprüche 7 bis 17 aktivierten NK-Zellen und gegebenenfalls eines wie in Anspruch 1 definierten Proteins, Fragmentes oder Derivats zur Herstellung eines Arzneimittels zur Behandlung von Tumoren, Krebserkrankungen, Infektionskrankheiten oder Autoimmunerkrankungen.

20. Verwendung nach Anspruch 19, wobei der Tumor ein solider Tumor oder eine Metastase ist.

21. Verwendung nach Anspruch 19, wobei die Krebserkrankung Leukämie oder ein Lymphom ist.

22. Verwendung nach Anspruch 19, wobei die Infektionskrankheit viralen, mykologischen oder bakteriellen Ursprungs ist.

23. Arzneimittel, Medizinprodukt oder medizinischer Hilfsstoff, das/der ein wie in Anspruch 1 definiertes C-terminales Fragment von Hsp70-Protein oder ein Derivat davon oder von nach dem Verfahren nach einem der Ansprüche 7 bis 17 aktivierten NK-Zellen in einer therapeutisch wirksamen Menge sowie gegebenenfalls übliche Träger- und/oder Hilfsstoffe enthält.

24. Arzneimittel, Medizinprodukt oder medizinischer Hilfsstoff nach Anspruch 23, wobei das Protein in einer Konzentration von mindestens 1 µg/ml, bevorzugt bis zu 1000 µg/ml, vorliegt.

25. Verwendung nach einem der Ansprüche 1 bis 6 oder 15 bis 22 oder Verfahren nach einem der Ansprüche 7 bis 17 oder Arzneimittel, Medizinprodukt oder medizinischer Hilfsstoff nach Anspruch 23 oder 24, wobei das Hsp70-Protein ein humanes Protein ist.

26. Verwendung nach einem der Ansprüche 1 bis 6, 15 bis 22 oder 25 oder Verfahren nach einem der Ansprüche 7 bis 17 oder 25 oder Arzneimittel, Medizinprodukt oder medizinischer Hilfsstoff nach einem der Ansprüche 23 bis 25, wobei das Hsp70-Protein oder dessen Fragment oder Derivat ein rekombinantes Protein ist.

27. Verwendung nach einem der Ansprüche 1 bis 6, 15 bis 22, 25 oder 26 oder Verfahren nach einem der Ansprüche 7 bis 17, 25 oder 26 oder Arzneimittel, Medizinprodukt oder medizinischer Hilfsstoff nach einem der Ansprüche 23 bis 26, wobei das C-terminale Fragment des Hsp70-Proteins die Aminosäuren 384 bis 561 von menschlichem Hsp70 oder den entsprechenden Bereich aus einem anderem Hsp70 umfasst, das die erfmdungsgemäßen Wirkungen aufweist.

28. Verwendung der nach einem Verfahren nach einem oder mehreren der vorhergehenden Ansprüche behandelten NK-Zellen zur Herstellung eines Arzneimittels zur Therapie von Tumorerkrankungen und/oder Infektionserkrankungen.

29. Verwendung nach Anspruch 28, wobei die Therapie durch Reinfundierung der behandelten NK-Zellen erfolgt.

## Claims

1. Use of a Hsp70 protein which is not complexed with peptides of tumor cells, a C-terminal fragment thereof or a derivative thereof or a protein with an amino acid sequence homology to the region of amino acids 384 to 641 of the Hsp70 protein of > 70% for the production of a pharmaceutical preparation, a medical product or a medical adjuvant for the treatment of tumors, cancer diseases or infectious diseases by activation of NK-cells.

2. Use of a Hsp70 protein which is not complexed with peptides of tumor cells, a C-terminal fragment thereof or a derivative thereof or a protein with an amino acid sequence homology to the region of amino acids 384 to 641 of the Hsp70 protein of > 70% for in vitro or ex vivo activation ofNK-cells.

3. Use according to claim 1 or 2, wherein the activation comprises the induction of an immune response mediated by NK-cells.

4. Use according to any one of claims 1 to 3, wherein the activation includes a stimulation of the proliferation of NK-cells and/or an increase of the cytolytic activity of NK-cells.

5. Use according to claim 4, wherein the cytolytic activity against tumor cells or infected cells of patients with infectious diseases is increased.

6. Use according to claim 5, wherein the cytolytic activity against leukaemia cells, lymphoma cells, tumor cells, metastasizing cells of solid tumors or cells of patients infected with viruses, bacteria and/or fungi is increased.

7. A method for ex vivo or in vitro activation of NK-cells, wherein a physiological cell suspension containing NK-cells is mixed and incubated with a protein, fragment or derivative as defined in claim 1 to effect an activation of the NK-cells.

8. The method according to claim 7, wherein the activation comprises a stimulation of the proliferation of the NK-cells and/or an increase of their cytotoxicity.

9. The method according to claim 7 or 8, wherein peripheral, mononuclear blood cells or a fraction containing NK-cells is used as physiological cell suspension containing NK-cells.

10. The method according to any one of claims 7 to 9, wherein the cell suspension further contains human or animal cells expressing Hsp70 on the cell surface.

11. The method according to claim 10, wherein tumor cells or infected cells of patients with infectious diseases are used as human or animal cells.

12. The method according to claim 11, wherein leukaemia cells, lymphoma cells, tumor cells, metastasizing cells of solid tumors or cells of patients infected with viruses, bacteria and/or fungi are used as human or animal cells.

13. The method according to any one of claims 7 to 12, wherein the physiological cell suspension containing the cells and proteins is incubated for at least 3 hours.

14. The method according to claim 13, wherein the incubation is carried out for 4 days.

15. Use according to any one of claims 1 to 6 or the method according to any one of claims 7 to 14, wherein a cytokine is used in addition.

16. Use or method according to claim 15, wherein an interleukin is used as cytokine.

17. Use or method according to claim 16, wherein IL-2, IL-12 or IL-15 is used as interleukin.

18. Use of a pharmaceutically effective amount of NK-cells activated according to the method according to any one of claims 7 to 17 for the production of a pharmaceutical preparation for the in vivo activation of the immune system, wherein said pharmaceutical preparation is administered optionally in combination with or before a pharmaceutically effective amount of a protein, fragment or derivative as defined in claim 1.

19. Use of a pharmaceutically effective amount of NK-cells activated according to the method according to any one of claims 7 to 17 and optionally a protein, fragment or derivative as defined in claim 1 for the production of a pharmaceutical preparation for the treatment of tumors, cancer diseases, infectious diseases or autoimmune diseases.

20. Use according to claim 19, wherein the tumor is a solid tumor or a metastasis.

21. Use according to claim 19, wherein the cancer disease is leukaemia or a lymphoma.

22. Use according to claim 19, wherein the infectious disease has a viral, mycological or bacterial origin.

23. Pharmaceutical preparation, medical product or medical adjuvant containing a C-terminal fragment of a Hsp70 protein as defined in claim 1 or a derivative thereof or NK-cells activated according to the method according to any one of claims 7 to 17 in a therapeutically effective amount as well as optionally common carrier substances and/or adjuvants.

24. The pharmaceutical preparation, medical product or medical adjuvant according to claim 23, wherein the protein is present in a concentration of at least 1 J.1g/ml, preferably up to 1000 µg/ml.

25. Use according to any one of claims 1 to 6 or 15 to 22 or the method according to any one of claims 7 to 17 or pharmaceutical preparation, medical product or medical adjuvant according to claim 23 or 24, wherein the Hsp70 protein is a human protein.

26. Use according to any one of claims 1 to 6, 15 to 22 or 25 or the method according to any one of claims 7 to 17 or 25 or pharmaceutical preparation, medical product or medical adjuvant according to any one of claims 23 to 25, wherein the Hsp70 protein or its fragment or derivative is a recombinant protein.

27. Use according to any one of claims 1 to 6, 15 to 22, 25 or 26 or the method according to any one of claims 7 to 17, 25 or 26 or pharmaceutical preparation, medical product or medical adjuvant according to any one of claims 23 to 26, wherein the C-terminal fragment of the Hsp70 protein comprises the amino acids 348 to 561 of human Hsp70 or the corresponding region of another Hsp70 comprising the effects of the invention.

28. Use of the NK-cells treated according to a method according to one or more of the preceding claims for the production of a pharmaceutical preparation for the therapy of tumor diseases and/or infectious diseases.

29. Use according to claim 28, wherein the therapy is carried out by re-infusion of the treated NK-cells.

## Revendications

1. Utilisation d'une protéine Hsp70, qui n'est pas complexée avec des peptides de cellules tumorales, d'un fragment C-terminal de celle-ci ou d'un dérivé de celle-ci ou d'une protéine avec une homologie de séquence d'acides aminés de la région des acides aminés 384 à 641 de la protéine Hsp70 supérieure à 70 % pour la fabrication d'une préparation pharmaceutique, d'une préparation médicinale ou d'un adjuvant médicinal pour le traitement de tumeurs, de cancers ou de maladies infectieuses par activation des cellules NK (cellules tueuses naturelles).

2. Utilisation d'une protéine Hsp70 qui n'est pas complexée avec des peptides de cellules tumorales, d'un fragment C-terminal de celle-ci ou d'un dérivé de celle-ci ou d'une protéine avec une homologie de séquence d'acides aminés de la région des acides aminés 384 à 641 de la protéine Hsp70 supérieure à 70 % pour l'activation in vitro ou ex vivo des cellules NK.

3. Utilisation selon la revendication 1 ou 2, dans laquelle l'activation comprend l'induction d'une réponse immunitaire médiée par des cellules NK.

4. Utilisation selon l'une quelconque des revendications 1 à 3, dans laquelle l'activation comprend la stimulation de la prolifération des cellules NK et/ou une augmentation de l'activité cytolytique des cellules NK.

5. Utilisation selon la revendication 4, dans laquelle l'activité cytolytique contre les cellules tumorales ou les cellules infectées des patients atteints de maladies infectieuses est augmentée.

6. Utilisation selon la revendication 5, dans laquelle l'activité cytolytique contre les cellules leucémiques, les cellules de lymphomes, les cellules tumorales, les cellules métastatiques de tumeurs solides ou les cellules de patients infectées avec des virus, des bactéries et/ou des champignons est augmentée.

7. Procédé d'activation ex vivo ou in vitro des cellules NK, dans lequel une suspension de cellules physiologiques contenant des cellules NK est mélangée et mise à incuber avec une protéine, un fragment ou un dérivé selon la revendication 1 afin de produire une activation des cellules NK.

8. Procédé selon la revendication 7, dans lequel l'activation comprend une stimulation de la prolifération des cellules NK et/ou une augmentation de leur cytotoxicité.

9. Procédé selon la revendication 7 ou 8, dans lequel des cellules sanguines mononucléaires périphériques ou une fraction contenant des cellules NK sont/est utilisée(s) comme suspension de cellules physiologiques contenant des cellules NK.

10. Procédé selon l'une quelconque des revendications 7 à 9, dans lequel la suspension cellulaire contient en plus des cellules humaines ou animales exprimant Hsp70 sur la surface des cellules.

11. Procédé selon la revendication 10, dans lequel on utilise comme cellules humaines ou animales, des cellules tumorales ou des cellules infectées de patients atteints de maladies infectieuses.

12. Procédé selon la revendication 11, dans lequel on utilise comme cellules humaines ou animales des cellules leucémiques, des cellules de lymphomes, des cellules tumorales, des cellules métastatiques de tumeurs solides ou des cellules de patients infectés avec des virus, des bactéries et/ou des champignons.

13. Procédé selon l'une quelconque des revendications 7 à 12, dans lequel la suspension de cellules physiologiques contenant les cellules et les protéines est mise à incuber pendant 3 heures au moins.

14. Procédé selon la revendication 13, dans lequel l'incubation est réalisée pendant 4 jours.

15. Utilisation selon l'une quelconque des revendications 1 à 6, ou procédé selon l'une quelconque des revendications 7 à 14, dans lesquels on utilise en plus une cytokine.

16. Utilisation ou procédé selon la revendication 15, dans lesquels une interleukine est utilisée comme cytokine.

17. Utilisation ou procédé selon la revendication 16, dans lesquels IL-2, IL-12 ou IL-15 est utilisée comme interleukine.

18. Utilisation d'une quantité pharmaceutiquement efficace de cellules NK activées selon le procédé selon l'une quelconque des revendications 7 à 17 pour la production d'une préparation pharmaceutique pour l'activation in vivo du système immunitaire, dans laquelle ladite préparation pharmaceutique est administrée facultativement en association avec, ou avant, une quantité pharmaceutiquement active d'une protéine, d'un fragment ou d'un dérivé tels que définis dans la revendication 1.

19. Utilisation d'une quantité pharmaceutiquement efficace de cellules NK activées selon le procédé selon l'une quelconque des revendications 7 à 17 et, facultativement, d'une protéine, d'un fragment ou d'un dérivé tels que définis dans la revendication 1, pour la production d'une préparation pharmaceutique pour le traitement de tumeurs, cancers, maladies infectieuses ou maladies auto-immunes.

20. Utilisation selon la revendication 19, dans lequel la tumeur est une tumeur solide ou une métastase.

21. Utilisation selon la revendication 19, dans lequel le cancer est une leucémie ou un lymphome.

22. Utilisation selon la revendication 19, dans lequel la maladie infectieuse est d'origine virale, mycologique ou bactérienne.

23. Préparation pharmaceutique, préparation médicinale ou adjuvant médicinal contenant un fragment C-terminal d'une protéine Hsp70 comme défini dans la revendication 1, ou d'un dérivé de celle-ci ou des cellules NK activées selon le procédé selon l'une quelconque des revendications 7 à 17 en quantité thérapeutiquement efficace, ainsi que, facultativement, des excipients et/ou adjuvants communs.

24. Préparation pharmaceutique, préparation médicinale ou adjuvant médicinal selon la revendication 23, dans lesquels la protéine est présente selon une concentration d'au moins 1 µg/ml, et préférablement allant jusqu'à 1000 µg/ml.

25. Utilisation selon l'une quelconque des revendications 1 à 6 ou 15 à 22, ou procédé selon l'une quelconque des revendications 7 à 17, ou préparation pharmaceutique, préparation médicinale ou adjuvant médicinal selon la revendication 23 ou 24, dans lesquels la protéine Hsp70 est une protéine humaine.

26. Utilisation selon l'une quelconque des revendications 1 à 6, 15 à 22, ou 25, ou procédé selon l'une quelconque des revendications 7 à 17, ou 25, ou préparation pharmaceutique, préparation médicinale ou adjuvant médicinal selon l'une quelconque des revendications 23 à 25, dans lesquels la protéine Hsp70 ou son fragment ou dérivé est une protéine recombinée.

27. Utilisation selon l'une quelconque des revendications 1 à 6, 15 à 22, 25 ou 26, ou procédé selon l'une quelconque des revendications 7 à 17, 25 ou 26, ou préparation pharmaceutique, préparation médicinale ou adjuvant médicinal selon l'une quelconque des revendications 23 à 26, dans lesquels le fragment C-terminal de la protéine Hsp70 comprend les acides aminés 348 à 561 de la Hsp70 humaine, ou la région correspondante d'une autre Hsp70 comprenant les effets de l'invention.

28. Utilisation des cellules NK traitées selon un procédé selon l'une ou plusieurs des revendications précédentes pour la production d'une préparation pharmaceutique pour la thérapie de cancers et/ou de maladies infectieuses.

29. Utilisation selon la revendication 28, dans laquelle la thérapie est réalisée par réinjection des cellules NK traitées.
